# EUROPEAN PATENT APPLICATION

(11) **EP 4 851 918 A1**
(43) Date of publication of application: **09.12.2026**
(21) Application number: 25305796.2
(22) Date of filing: 02.06.2025
(51) Int. Cl.: G06T 7/38

(54) **ALIGNEMENT OF A FIRST AND SECOND STACK OF SLICES**

(71) Applicant: Dassault Systèmes, 78140 Vélizy-Villacoublay (FR)
(72) Inventor: BORNACHOT, Yohan, 78946 Vélizy-Villacoublay (FR)
(74) Representative: Bandpay & Greuter

(57) **Abstract**

The disclosure notably relates to a computer-implemented method for aligning a first and a second stack of slices along a common reference axis normal to each slice of the first and second stacks. The method comprises obtaining the first and the second stack. Each stack has a predetermined interslice space. The method also comprises computing, for each stack, a set of coordinates. The method also comprises computing an intersection between the two sets. The method also comprises creating, for each stack, a new physical scale of coordinates sharing a common origin. The method also comprises computing, for each stack, the mean of the new physical scale and subtracting the mean computed for the second stack to the mean computed for the first stack. The method also comprises aligning the first and the second stacks of slices by offsetting the second stack of slices with the obtained vertical offset.

## Description

### TECHNICAL FIELD

The disclosure relates to the field of computer programs and systems, and more specifically to a method, system and program for aligning a first and a second stack of slices along a common reference axis.

### BACKGROUND

Image registration techniques have been gaining more and more interest for comparing or integrating sets of data. Image registration refers to the process of spatially transforming different sets of data into one common coordinate system. Said otherwise, the registration is the process of transforming different sets of images into a common coordinate system, so that they can be compared accurately. The sets of data may comprise, e.g., slices consisting of photographs, images and/or data obtained (e.g., by sensors) from different time points, viewpoints. Image registration techniques have been proven useful for several applications in the field of computer vision and has also found its way into the aerospace field (for example for compiling data from satellites) and in the field of medical imaging.

Regarding two medical images, the goal of registration is to find the spatial transformation that maximizes the similarity between the images, thereby ensuring that corresponding features in the images are correctly aligned.

The alignment of the sets of data is of an important matter in the context of image registration. Indeed, the alignment plays a crucial role in convergence towards the optimal transformation. The alignment of the sets of data is also useful for visualization of the sets of data, e.g., when it is necessary to align several slices of medical images with each other as in the case of patients undergoing longitudinal studies.

Within this context, there is still a need for an improved method for aligning a first and a second stack of slices along a common reference axis.

### SUMMARY

It is therefore provided a computer-implemented method for aligning a first and a second stack of slices along a common reference axis. Each slice is a medical image. The common reference axis is normal to each slice of the first and second stacks. The method comprises obtaining the first and the second stack of slices. Each stack has a predetermined interslice space. The method also comprises computing, for each stack, a set of coordinates. The set of coordinates lie in a scale common to the first and second stacks. Each coordinate is associated, in the common reference axis, with a slice. The method also comprises computing an intersection between the two sets of coordinates. The method thereby identifies overlapping slices having coordinates which lie in the intersection. The method also comprises creating, for each stack, a new physical scale of coordinates. The new physical scales of coordinates share a common origin. Two consecutive coordinates of each new physical scale are spaced by the predetermined interslice space. The method also comprises computing, for each stack, the mean of the new physical scale of coordinates. The mean is associated to overlapping slices. The method also comprises subtracting the mean computed for the second stack to the mean computed for the first stack. The method thereby obtains a vertical offset along the common reference axis. The method also comprises aligning the first and the second stacks of slices by offsetting the second stack of slices with the obtained vertical offset.

The method may comprise one or more of the following:
- computing the set of coordinates comprises:
   ∘ obtaining a trained neural network, the neural network being configured to take as input a pair of stacks of slices and to output a set of coordinates lying in a scale common to the pair of stacks, each coordinate being associated, in the common reference axis, with a slice;
   o inputting the first and the second stack of slices to the trained neural network; and
   ∘ outputting the set of coordinates from the neural network;
- the trained neural network has been trained according to a machine-learning method comprising:
   o providing a dataset of stacks of slices; and
   o training the neural network based on the dataset, the training comprising providing as input a pair of stacks of slices from the dataset and regressing a slice score for each slice of the stack;
- regressing the slice score comprises minimizing a loss, the loss rewarding, for each slice score of a respective slice of the stack, at least one of:
   ∘ an ordering of each slice score;
   ∘ attributing a regular spacing between pairs of slice scores;
   ∘ resembling to straight lines;
- creating, for each stack, the new physical scale of coordinates sharing the common origin comprises setting a respective set of new coordinates, a new coordinate of the respective set corresponding to the common origin added to an index of a respective slice of the stack multiplied by an interslice space for the stack;
- setting the set of coordinates is performed, for a respective slice of each stack according to the formula: $∀ i ∈ \left[1, {N}_{1}\right] , {z}_{i} = {z}_{0} + i \times {δ}_{{z}_{1}}$ $∀ j ∈ \left[1, {N}_{2}\right] , {z}_{j} = {z}_{0} + j \times {δ}_{{z}_{2}}$ wherein *N*₁, *N*₂ denote respectively the number of slices in the first stack and the second stack, i denotes an index of a slice of the first stack, j denotes an index of a slice of the second stack, *z*₀ denotes the common origin of the physical scale for each volume, and *δ*_{*z*₁},*δ*_{*z*₂} denote respectively an interslice space for the first stack and the second stack;
- computing the mean of the new physical scale of coordinates for each stack is performed according to the formula: $\overline{{Z}_{1}} = \frac{1}{Card \left({O}_{1}\right)} {\sum }_{{z}_{i} ∈ {O}_{1}} {z}_{i}$ $\overline{{Z}_{2}} = \frac{1}{Card \left({O}_{2}\right)} {\sum }_{{z}_{i} ∈ {O}_{2}} {z}_{i}$ wherein Z̅₁̅ denotes the mean of the new scale of coordinates for the first stack, Z̅₂̅ denotes the mean of the new scale of coordinates for the second stack, *O*₁ denotes the set of new coordinates for the new physical scale of coordinates for the first stack and *O*₂ denotes the set of new coordinates for the new physical scale of coordinates for the second stack;
- obtaining the vertical offset along the common reference axis is performed according to the formula: $Δ {Z}_{2 \to 1} = \overline{{Z}_{1}} - \overline{{Z}_{2}} ,$
- wherein Δ*Z*_{2→1} denotes the vertical offset;
- further comprising applying a vertical translation to the slices of the first stack and the second stack, the vertical translation having a norm equal to the vertical offset;
- each slice is a CT-Scan or an MRI or a PET Scan or SPECT Scan; and/or
- further comprising displaying and/or storing the aligned first and the second stacks of slices.

It is further provided a computer program comprising instructions which, when the program is executed by a computer, cause the computer to carry out the method.

It is further provided a computer readable storage medium having recorded thereon the computer program.

It is further provided a system comprising a processor coupled to a memory, the memory having recorded thereon the computer program.

### BRIEF DESCRIPTION OF THE DRAWINGS

Non-limiting examples will now be described in reference to the accompanying drawings, where:
- FIG. 1 shows a flowchart of an example of the method;
- FIG. 2 shows an example of the system; and
- FIG.s 3 to 8 illustrate examples of the method.

### DETAILED DESCRIPTION

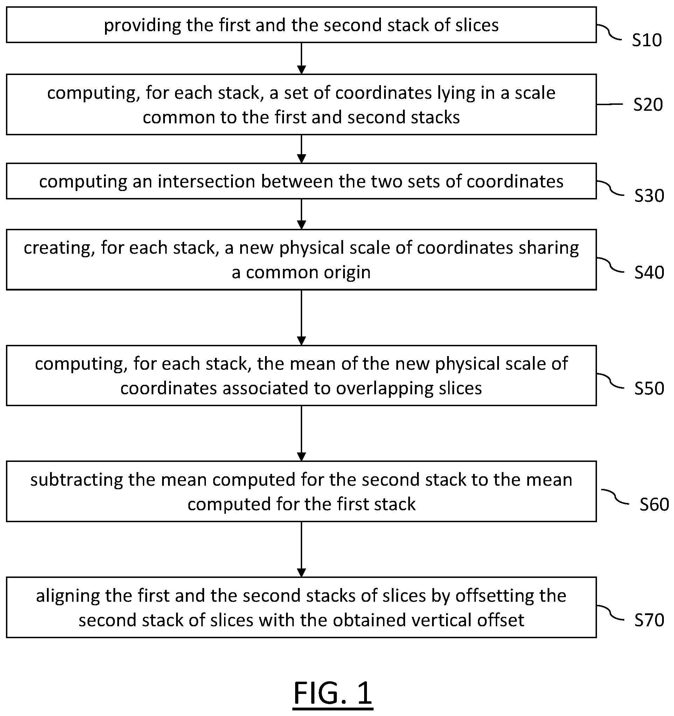

With reference to the flowchart of FIG. 1, it is proposed a computer-implemented method for aligning a first and a second stack of slices along a common reference axis. Each slice is a medical image. The common reference axis is normal to each slice of the first and second stacks. The method comprises obtaining the first and the second stack of slices. Each stack has a predetermined interslice space. The method also comprises computing, for each stack, a set of coordinates. The set of coordinates lie in a scale common to the first and second stacks. Each coordinate is associated, in the common reference axis, with a slice. The method also comprises computing an intersection between the two sets of coordinates. The method thereby identifies overlapping slices having coordinates which lie in the intersection. The method also comprises creating, for each stack, a new physical scale of coordinates. The new physical scales of coordinates share a common origin. Two consecutive coordinates of each new physical scale are spaced by the predetermined interslice space. The method also comprises computing, for each stack, the mean of the new physical scale of coordinates. The mean is associated to overlapping slices. The method also comprises subtracting the mean computed for the second stack to the mean computed for the first stack. The method thereby obtains a vertical offset along the common reference axis. The method also comprises aligning the first and the second stacks of slices by offsetting the second stack of slices with the obtained vertical offset.

Such a method improves the alignment of the first and the second stack of slices along the common reference axis. Notably, the alignment is performed based on overlapping slices having coordinates which lie in the intersection, which ensures that the alignment is focused on anatomical relevant portions, i.e., that the slices are representative of a common overlap, e.g., on a same portion of a patient's body. Moreover, as the method creates the new physical scales, the method ensures that the alignment shows physically accurate spacing between the slices.

Furthermore, the alignment of the first and second stack of slices enables an efficient visual comparison for identifying differences between the slices of the first stack and the slices of the second stack. This is all thanks to the alignment taking into account the physical scale of coordinates sharing the common origin, which enable to identify variations in physical space represented by respective slices of the first and second stack, e.g., slices representing the evolution of a patient at different times.

The alignment of the first and second stacks is particularly advantageous for performing image registration. It has been found that the alignment provides an efficient initialization prior to spatially transforming the first stack and the second stack into one common coordinate system. Indeed, knowing the vertical offset is a better initialization than, e.g., sampling random parameters or aligning volume centers for initializing the transformation, e.g., before an optimization process.

The method is computer-implemented. This means that steps (or substantially all the steps) of the method are executed by at least one computer, or any system alike. Thus, steps of the method are performed by the computer, possibly fully automatically, or, semi-automatically. In examples, the triggering of at least some of the steps of the method may be performed through user-computer interaction. The level of user-computer interaction required may depend on the level of automatism foreseen and put in balance with the need to implement user's wishes. In examples, this level may be user-defined and/or pre-defined.

A typical example of computer-implementation of a method is to perform the method with a system adapted for this purpose. The system may comprise a processor coupled to a memory and a graphical user interface (GUI), the memory having recorded thereon a computer program comprising instructions for performing the method. The memory may also store a database. The memory is any hardware adapted for such storage, possibly comprising several physical distinct parts (e.g. one for the program, and possibly one for the database).

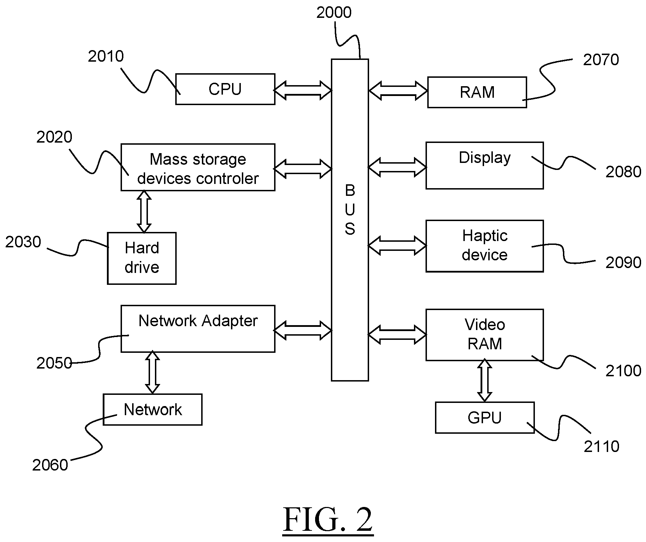

FIG. 2 shows an example of the system, wherein the system is a client computer system, e.g. a workstation of a user.

The client computer of the example comprises a central processing unit (CPU) 2010 connected to an internal communication BUS 2000, a random access memory (RAM) 2070 also connected to the BUS. The client computer may be further provided with a graphical processing unit (GPU) 2110 which is associated with a video random access memory 2100 connected to the BUS. Video RAM 2100 is also known in the art as frame buffer. The client computer may be further provided with a mass storage device controller 2020 manages accesses to a mass memory device, such as hard drive 2030. Mass memory devices suitable for tangibly embodying computer program instructions and data include all forms of nonvolatile memory, including by way of example semiconductor memory devices, such as EPROM, EEPROM, and flash memory devices; magnetic disks such as internal hard disks and removable disks; magneto-optical disks. Any of the foregoing may be supplemented by, or incorporated in, specially designed ASICs (application-specific integrated circuits). A network adapter 2050 manages accesses to a network 2060. The client computer may also include a haptic device 2090 such as cursor control device, a keyboard or the like. A cursor control device is used in the client computer to permit the user to selectively position a cursor at any desired location on display 2080. In addition, the cursor control device allows the user to select various commands, and input control signals. The cursor control device includes a number of signal generation devices for input control signals to system. Typically, a cursor control device may be a mouse, the button of the mouse being used to generate the signals. Alternatively or additionally, the client computer system may comprise a sensitive pad, and/or a sensitive screen.

The computer program may comprise instructions executable by a computer, the instructions comprising means for causing the above system to perform the method. The program may be recordable on any data storage medium, including the memory of the system. The program may for example be implemented in digital electronic circuitry, or in computer hardware, firmware, software, or in combinations of them. The program may be implemented as an apparatus, for example a product tangibly embodied in a machine-readable storage device for execution by a programmable processor. Method steps may be performed by a programmable processor executing a program of instructions to perform functions of the method by operating on input data and generating output. The processor may thus be programmable and coupled to receive data and instructions from, and to transmit data and instructions to, a data storage system, at least one input device, and at least one output device. The application program may be implemented in a high-level procedural or object-oriented programming language, or in assembly or machine language if desired. In any case, the language may be a compiled or interpreted language. The program may be a full installation program or an update program. Application of the program on the system results in any case in instructions for performing the method. The computer program may alternatively be stored and executed on a server of a cloud computing environment, the server being in communication across a network with one or more clients. In such a case a processing unit executes the instructions comprised by the program, thereby causing the method to be performed on the cloud computing environment.

The method is for aligning a first and a second stack of slices along a common reference axis. In other words, slices of the first and second stack are arranged so as to match (approximately or substantially) spatially in the common reference axis. For example, a respective slice of the first stack and a respective slice of the second stack may represent a same spatial location in the common reference axis.

The common reference axis may be a reference line in a coordinate frame (e.g., a Cartesian coordinate frame) for locating coordinates for slices of the first and second stacks. The common reference axis may be based on anatomical information, e.g., based on the length of the human body when viewed from an anterior view (in other words, a frontal view). For example, the common reference axis may extend from a location at a lower portion of the body (e.g., the legs of the body) to a location at an upper portion of the body (e.g., the chest, neck or head). The common reference axis may be oriented, e.g., from the location at the lower portion of the body to the location at the upper portion of the body. The orientation may be set according to any convention. For example, the common reference axis may have a value of zero at the location at the lower portion of the body and a value of one at the location of the upper portion of the body. The common reference axis is normal to each slice of the first and second stacks. In other words, coordinates associated to each slice of the first and second slice may have values between zero and one.

The method comprises obtaining S10 the first and the second stack of slices.

Obtaining S10 the first and the second stack of slices may comprise retrieving such stacks from a non-volatile storage, or retrieving them from a network. The first stack and the second stack may be obtained at different times, by different persons/entities. The second stack may be obtained at a time after the first stack. In other words, the slices of the first stack may be obtained at a time preceding the time of the slices of the second stack. In such a case the first stack may be called "baseline" stack and the second stack may be called "follow-up" stack. The slices of the second stack may stem from a medical imaging performed during a follow up procedure of the patient, e.g., at a time period (e.g., days or months) after the slices of the first stack where obtained.

As known in the art, the baseline set (i.e., the first stack) may refer to the initial set of medical images taken of the lesion, e.g. at the starting point of observation or treatment. As known in the art, the follow-up set (i.e., the second stack) of images may be subsequent medical images taken after the baseline images, at later time points, to monitor the evolution or response of a lesion, condition, or treatment over time.

Each slice of the first stack and the second stack is a medical image. The slices of the first stack (respectively the second stack) may represent a portion of a human body tissue. The slices of the second stack may represent the same portion of the human body tissue represented by the first stack. A tissue may be a group of cells, e.g., in close proximity, organized to perform one or more specific functions, as known in the art. The human body tissue may thus correspond to an organ (such as the liver, lungs, heart or bones) or may correspond to a tissue corresponding to a portion of the organ.

In examples, each slice (of the first stack and/or the second stack) may be a CT-Scan. Alternatively, each scan may be an MRI. Alternatively, each scan may be a PET Scan. Alternatively, each scan may be a SPECT Scan. A CT-scan (Computed Tomography Scan) is a specific type of medical images produced by sending X-rays through the human body. The signal is read and analyzed to reconstruct a dense volume of the body. Practically, the volume is split into slices whose normal direction is feet to head. An MRI (Magnetic Resonance Imaging) is a specific type of medical images technique that uses a magnetic field and computer-generated radio waves to create detailed images of the organs and tissues in the body. PET-scan (Positron Emission Tomography) is an imaging test that can help reveal the metabolic or biochemical function of your tissues and organs. The PET scan uses a radioactive drug called a tracer to show both typical and atypical metabolic activity. A SPECT Scan is the result of a type of nuclear imaging test that provides 3D images of internal organs and tissues. SPECT-Sanning may use a Gamma camera and a radioactive tracer to evaluate how blood flows to tissues and organs, as well as to assess their function.

It is to be understood that any non-invasive technology for obtaining 2D or 3D cross-sectional images with imaging modality offering contrast between different types of tissues or regions of interest can be used. In examples, obtaining the first and second stacks may comprise performing a medical imaging on a patient. In other words, the slices of the first stack (respectively second stack) may stem from a medical imaging performed on an anatomical region of a patient enclosing a portion of the human body tissue at a given time. The medical imaging may be, for example, CT-Scanning, Medical Resonance Imaging - MRI, Positron Emission Tomography -PET Scanning or Single photon emission computed tomography - SPECT scanning.

The first and/or second stack may be ordered. For example, the slices may be obtained according to a position in which the portion of the human body tissue is viewed, e.g., from an axial view, for example from a lower location of the portion of the human body tissue represented by the stack to an upper location of the portion of the human body tissue. The first stack of images may consist of slices being medical images each of a (same) view of the portion of the human body tissue on a plane such as x-y plane (also called axial plane of the body). A given stack (e.g., the first and/or second stack) may sample the portion of the human body tissue along the z-axis. The plane may be chosen by convention, for example the view on the plane may be an axial (also called horizontal or transversal) view, sagittal (also called longitudinal) view or frontal (also called coronal) view.

Each stack (e.g., the first and/or second stack) has a predetermined interslice space. By "interslice space" it is meant the distance by which two consecutive slices (as found in the ordered stack) are separated over the portion of the human body tissue that is represented by each slice. The predetermined interslice space may be uniform, i.e., two consecutive slices of a stack are separated by the same distance. Alternatively, the predetermined interslice space may be not uniform, which case two consecutive slices of a stack may be separated by different distances.

In examples, the first and second stacks are of CT-scans. Each of the stacks may be ordered vertically, that is, from the lower portion of the body (e.g., the feet) to an upper part of the body (e.g., the head). In other words, each CT-scan is a medical image that refers to an anatomical location in human body, ordered vertically. In examples, the CT-scans may be associated to CT-scan file metadata. The CT-scan file metadata may comprise axial coordinates corresponding to each slice. The predetermined interslice space may be distance between axial coordinates (in terms of the axial axis) of two consecutive slices.

The method comprises computing S20, for each (first and second) stack, a set of coordinates. In other words, the method assigns a coordinate for each slice of a respective stack. Each coordinate of the set may be associated with a respective slice and may represent the location of the respective slice along the common reference axis. The common reference axis may be a standard axis, e.g., following a vertical axis, a horizontal axis or other anatomical axis such as an axis orthogonal to an axial view plane (similarly a sagittal view plane or a frontal view plane).

The set of coordinates lies in a scale common to the first and second stacks. Each coordinate is associated, in the common reference axis, with a slice. The scale may be a mapping onto the common reference axis. The mapping may take into account the ordering of the stacks into the common reference axis. In other words, the scale maps a respective slice into a position relative to the common reference axis. The scale may have a predetermined spacing between two consecutive coordinates. In other words, the scale may be seen as setting a grid of units into which the common reference axis is divided. Each unit of the grid may be limited by two consecutive coordinates.

In examples, computing S20 the set of coordinates may comprise obtaining a trained neural network. This means that the computerized system performing the method can access directly the neural network (e.g. the neural network runs onto the computerized system or is executed by the computerized system) or indirectly the neural network (e.g. the computerized system can provide an input to the neural network and obtain the output of the neural network obtained for the input).

The neural network may be configured to take as input stack of slices. The neural network may also be configured to output a set of coordinates lying in a scale common to the pair of stacks. In other words, the neural network may be configured for computing a location (determined by the coordinates) of the pair of slices based on the scale common to the pair of stacks. The scale may define the distance between each coordinate in the common reference axis. The neural network may be a function comprising a collection of connected nodes, also called "neurons". Each neuron receives an input and outputs a result to other neurons connected to it. The neurons and the connections linking each of them have weight values, which are adjusted via a training.

In examples, computing S20 the set of coordinates may also comprise inputting the first and the second stack of slices to the trained neural network. Computing S20 the set of coordinates may also comprise outputting the set of coordinates from the neural network. The set comprises vectors of coordinates, for the first and second stacks, lying in the scale common to the first and second stacks. In other words, for each slice of a respective stack, the neural network outputs a coordinate associated to the slice lying in the scale common to the first and second stacks. The result for a stack of slices is then a vector of coordinates.

In examples, the trained neural network may have been trained according to a machine-learning method. In the context of the present disclosure, by "trained neural network", it is meant that weights of the neural network are definitively saved after performing a learning/training on a dataset.

In examples, the machine-learning method may comprise providing a dataset. The dataset may comprise stacks of slices. The machine-learning method may also comprise training the neural network based on the dataset. The dataset may comprise stacks of slices wherein the slices are medical images. The training may comprise providing as input a pair of stacks of slices from the dataset and regressing a slice score for each slice of the stack. In examples, the regression may be performed via backpropagation.

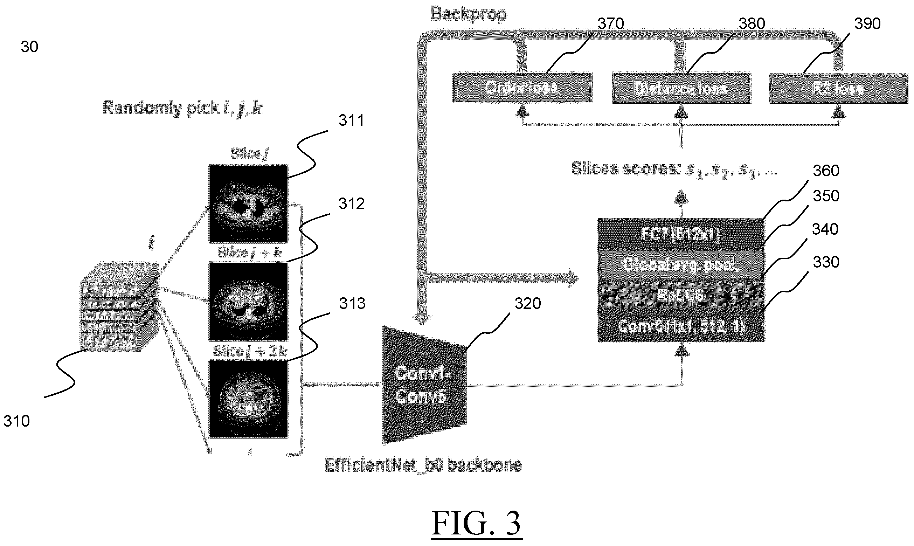

Reference is made to FIG. 3, showing a schematic of an example of a training flow 300 for the neural network.

In examples, the training may be performed by:
- providing the dataset of stacks of slices. The slices may be CT-scans, e.g., representing a desired anatomical coverage of the human body tissue;
- uniformly choosing a stack i 310, a starting slice *j* 311 (from the stack i), and a step between slices k;
- sampling the chosen stack i by selecting a slice each *k* slices (e.g., the slice with index *j +k* 312 and the stack *j*+ 2*k* 313), starting with the slice with index j 311, until arriving to the desired number of slices in each stack 310, denoted as *n_slices_per_sample;* and
- feeding the sampled slices to the neural network to regress slice scores one for each slice.

In the example of FIG. 3, the neural network comprises a backbone responsible for extracting semantically relevant features maps, and additional head layers allow the regression of slice scores. The slices 311-313 are input to the neural network via convolutional blocks 320. The neural network may also comprise layers Conv6(1x1,512,1) 330, ReLU6 340, a Global averaging pool 350 and FC7(512x1) 360. The layers output slice scores which are minimized with a loss, as explained below.

Preferably, the scores may be regularly spaced, and correctly ordered as slices are also sampled regularly.

In examples, regressing the slice score may comprise minimizing a loss. The loss may reward, for each slice score of a respective slice of the stack, at least one of an ordering of each slice score (e.g., denoted as an order loss term 370), attributing a regular spacing between pairs of slice scores (e.g., denoted as an distance loss term 380), and/or resembling to straight lines (e.g., denoted as an R2 loss term 370).

By rewarding an ordering of each slice score, it is meant that the loss penalizes the neural network for not ordering slices correctly. In other words, the loss penalizes that a higher slice (in the order set by the stack of slices) received a lower score than a lower slice. By rewarding a regular spacing between pairs of slice scores, it is meant that the loss penalizes the network for not attributing regularly spaced slice scores, that is, for two consecutive slices, the difference in slice score should be uniform. By rewarding resembling to straight lines, it is meant that the neural network penalizes slice scores output by the neural network that do not fit a straight line.

The trained neural network thus outputs a common axial scale of normalized coordinates (in the form of the set of coordinates) for which a specific range of coordinates always refer to the same anatomical area.

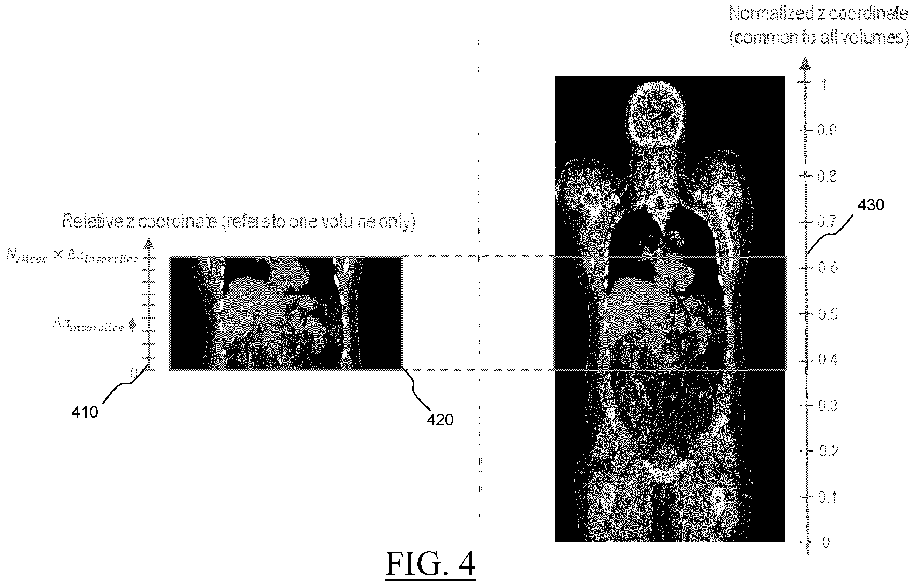

FIG. 4 illustrates examples of the input to the neural network and the output of the neural network.

The input is a stack of slices (medical CT-scans) showing a relative vertical scale, i.e., a relative z coordinate 410. The z coordinate 410 shows N slices sampling the anatomical region 420. Each slice is separated by an interslice space noted Δ*z_{interslice}.*

The output of the neural network is the set of coordinates 430 lying in a scale common to the first and second stacks. The set of coordinates 430 is a normalized z coordinate, i.e., the coordinates have values between 0 and 1. In this example, one out of ten slices is displayed.

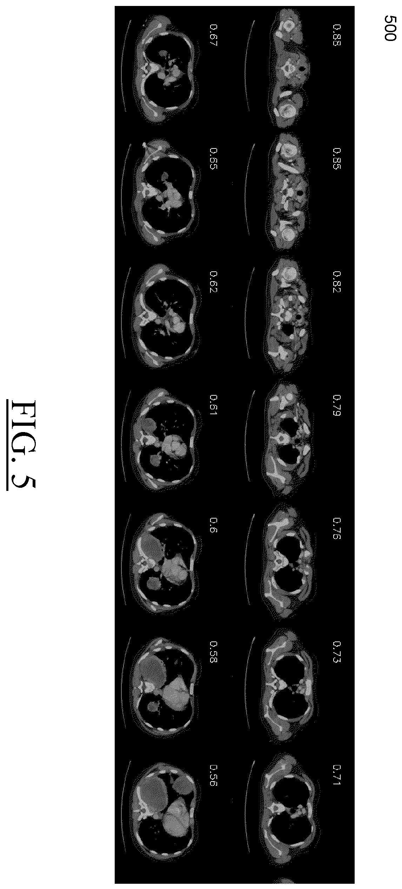

FIG. 5 illustrates examples of the slices and its associated coordinates of the set.

FIG. 5 shows fourteen slices 500. Each slice is associated with a coordinate ranging from 0.56 (thereby being located at the lowest position with respect to the normalized z coordinate) to 0.88 (thereby being located at the lowest position with respect to the normalized z coordinate).

The method comprises computing S30 an intersection between the two sets of coordinates, output at S20. That is, the method identifies coordinates associated to slices of the first and second stacks which are proximate along the common reference axis. The method thereby comprises identifying overlapping slices having coordinates which lie in the intersection. In other words, the method identifies slices from the first and second stacks which enclose a volume that jointly represents the same anatomical region. Steps S40 to S70 may thus be performed with respective subsets of the two sets which comprise the coordinates associated to the identified overlapping slices.

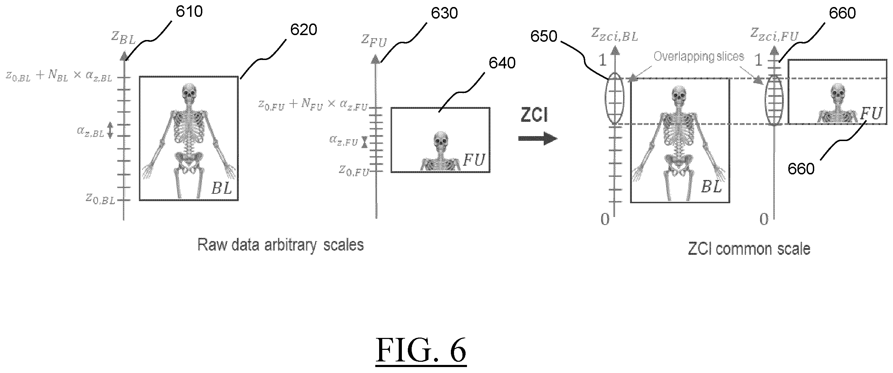

FIG. 6 illustrates an example of the intersection between the two sets of coordinates.

FIG. 6 shows a first stack *BL* (also called "baseline stack") and a second stack *FU* (also called "follow-up stack"). The first stack samples a region 620 of the human body. The slices of the first stack are ordered and shown with an axis *Z_{BL}.* The second stack samples a region 640 of the human body. The slices of the second stack are ordered and shown with an axis *Z_{FU}*. The intersection of the overlapping vertices is determined from the set of coordinates computed at S20. As the intersection takes into account the common reference axis, the method only takes slices 650 from the first stack which overlap on the same anatomical region as the slices of the second stack 660.

The method comprises creating S40, for each stack, a new physical scale of coordinates. The new physical scale is a new mapping, for a respective first or second stack, for coordinates that takes into account the physical distribution of the slices in the real world. In other words, the predetermined spacing between two consecutive coordinates of the physical scale is separated with a distance based on the physical separation of the portion of the human body represented by the slices of the respective first or second stack. The new physical scale of coordinates, of each stack, share a common origin. In other words, the origin of both new physical scales of coordinates overlaps. Two consecutive coordinates of the new physical scales (of each stack) are spaced by the predetermined interslice space. In other words, the two consecutive coordinates of the new physical scales are spaced based on the physical separation set by the predetermined interslice space.

In the context of CT-Scan slices, each slice is a medical image that refers to an anatomical location in human body, ordered vertically. Most part of the time axial coordinates corresponding to each slice are provided in the CT-scan file metadata. However, the axial coordinates are relative and proper to their respective stack. Based on this input, the method creates a common vertical scale that is shared by the first and second stacks, allowing the alignment of the stacks for visual comparison and medical follow up.

In examples, computing S40, for each stack, the new physical scale of coordinates sharing the common origin may comprise setting a respective set of new coordinates. In other words, the creation transforms the subset of coordinates of both stacks which lie in the intersection into a new position on the common reference axis that takes into account the predetermined interslice space. A new coordinate of the respective set may correspond to the common origin added to an index of a respective slice of the stack multiplied by an interslice space for the stack.

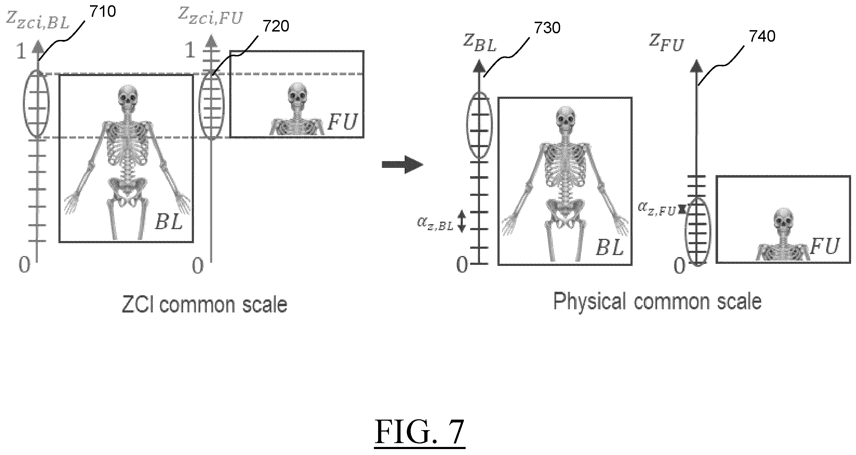

FIG. 7 illustrates the new set of physical coordinates.

FIG. 7 shows the slices 710 of the first stack *BL* intersecting with the slices 720 of the second stack *FU.* The new physical scales 730 and 740 are obtained by adding the common origin added to the index of the respective (of the first stack *BL* or the second stack *FU*) slice of the stack multiplied by an interslice space for the stack.

In examples, setting the respective set of coordinates may performed, for a respective slice of each stack according to the formula: $∀ i ∈ \left[1, {N}_{1}\right] , {z}_{i} = {z}_{0} + i \times {δ}_{{z}_{1}}$ $∀ j ∈ \left[1, {N}_{2}\right] , {z}_{j} = {z}_{0} + j \times {δ}_{{z}_{2}}$ wherein *N*₁, *N*₂ denote respectively the number of slices in the first stack and the second stack, *i* denotes an index of a slice of the first stack, *j* denotes an index of a slice of the second stack, *z*₀ denotes the common origin of the physical scale for each volume, and *δ*_{*z*₁}, *δ*_{*z*₂} denote respectively an interslice space for the first stack and the second stack.

The method comprises computing S50, for each stack, the mean of the new physical scale of coordinates associated to overlapping slices. The mean of the new physical scale for each stack may be the summation of coordinates in the set divided by the cardinality of the set of coordinates.

In examples, computing (S50) the mean of the new physical scale of coordinates for each stack may be performed according to the formula: $\overline{{Z}_{1}} = \frac{1}{Card \left({O}_{1}\right)} {\sum }_{{z}_{i} ∈ {O}_{1}} {z}_{i}$ $\overline{{Z}_{2}} = \frac{1}{Card \left({O}_{2}\right)} {\sum }_{{z}_{i} ∈ {O}_{2}} {z}_{i}$

*̅Z̅*̅₁̅ denotes the mean of the new scale of coordinates for the first stack. *̅Z̅*̅₂̅ denotes the mean of the new scale of coordinates for the second stack. *O*₁ denotes the set of new coordinates for the new physical scale of coordinates for the first stack and *O*₂ denotes the set of new coordinates for the new physical scale of coordinates for the second stack.

In other words, for the first stack, *̅Z̅*̅₁̅ denotes the summation of coordinates in the set of coordinates *O*₁, denoted as Σ*_{zi}* ∈ *O*₁ *zᵢ*, divided by the cardinality of the set of coordinates *Card*(*O*₁)*.* For the second stack, *̅Z̅*̅₂̅ denotes the summation of coordinates in the set of coordinates O₂, denoted as Σ*_{zi}* ∈ *O*₂ *zᵢ,* divided by the cardinality of the set of coordinates *Card*(*O*₂)*.*

The method comprises subtracting S60 the mean *̅Z̅*̅₁̅ of the new physical scale of coordinates for the second stack to the mean *̅Z̅*̅₂̅ of the new physical scale of coordinates computed for the first stack. The method thereby comprises obtaining a vertical offset along the common reference axis. In other words, the vertical offset represents the spatial displacement along the common reference axis between corresponding slices of the first and second stacks.

Obtaining the vertical offset along the common reference axis may be performed according to the formula: $Δ {Z}_{2 \to 1} = \overline{{Z}_{1}} - \overline{{Z}_{2}} .$

Δ*Z*_{2→1} denotes the vertical offset. *̅Z̅*̅₁̅ denotes the mean of the new scale of coordinates for the first stack computed at S50. *̅Z̅*̅₂̅ denotes the mean of the new scale of coordinates for the second stack computed at S50.

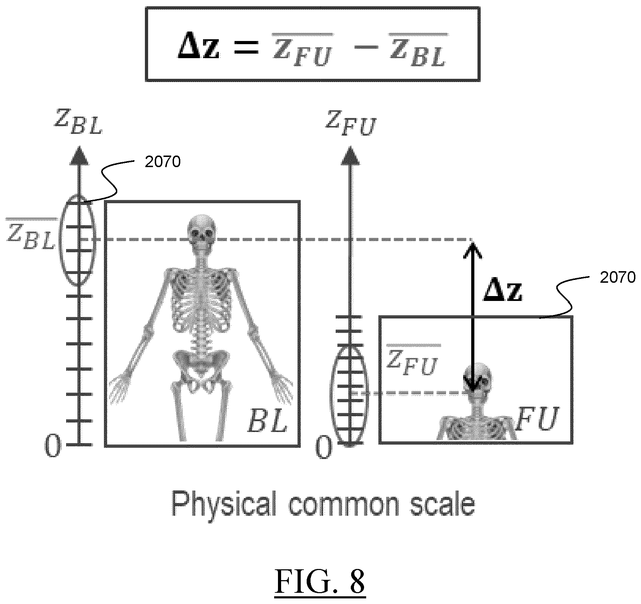

FIG. 8 illustrates the subtraction of the means. In other words, the difference between mean coordinates for the overlapping coordinates.

The method comprises aligning S70 the first and the second stacks of slices by offsetting the second stack of slices with the obtained vertical offset. In other words, the aligning S70 adjusts the spatial displacement along the common reference axis between corresponding slices of the first and second stacks. For example, the set of coordinates of the slices of the second stack may be subtracted with the vertical offset.

The method thus improves the alignment of the first and the second stack of slices along the common reference axis. Indeed, the set of coordinates computed at S20 serves as a standardized axis used for aligning and the first and second stacks. Thus, the first and second stacks are interpreted by the method within the same spatial distribution/orientation.

In addition, thanks to the use of the neural network, the method leverages from the neural network paradigm in order to obtain an accurate alignment. This is because the neural network is trained based on a dataset of stack of slices. Thus, the method learns from the variation of anatomical regions represented/captured by each stack, and thus the method can learn from such variability so as to determine sets of coordinates on a common scale, as the common scale can be set uniformly, independently of the anatomical region captured by the first and second stacks.

The computed intersection S30 ensures that the method focuses on subsets of the first and second stacks which share the same anatomical region, thereby allowing the method to align slices where there may be differences, for example where there is an evolution of the human body tissue represented in the anatomical region, e.g., where there is an evolution of a lesion or tumor.

The new physical scales created at S40 ensures that both stacks share a common origin, providing a consistent reference point for alignment. In addition, by spacing the coordinates with the interslice space, the new physical scales accurately reflect the spatial relationship between slices within each stack with respect to their anatomical context. Hence, the method enables precise alignment and comparison of the stacks along the common reference axis, while accounting for differences in their original physical parameters.

The computation of the mean S50 for each stack and the subtraction S60 enables the method to correct for differences in the positioning of the slices of the first and second stacks, so that the alignment S70 ensures that the aligned first and second stacks represent the same part of the human body, thereby enabling an improved visual comparison of the change in a tissue of the human body (e.g., a lesion) present in slices of the second stack relative to the slices of the first stack. Consequently, the method compensates the differences in alignment due to, e.g., patient positioning, differences in the acquisition set up such as acquisition angles or different imaging modalities.

In examples, the method may further comprise applying a vertical translation. In other words, the method may adjust a translation of the coordinates of the first and second stack along the common reference axis. The vertical translation may be applied to the slices of the first stack and the second stack. The vertical translation may have a norm equal to the vertical offset. In other words, the vertical translation applied to each slice (associated to a corresponding coordinate) of the first and second stack is displaced with a magnitude equal to the vertical offset.

The vertical translation thereby serves for smart registration initialization for further multi-directional alignment between volumes. Hence, the method is faster compared to brute force grid search for finding the best translation before optimizing registration, for example when using Fast Translation Alignment (FASTA).

In examples, the method may further comprise displaying the aligned first and second stacks of slices. The method may comprise rendering the aligned slices on a display device. The displaying thereby enables the comparison of the aligned stacks. The method may further comprise user-interaction when displaying the aligned first and second stacks, for example zooming, panning, or changing the color of the slices. The method thus enhances user analysis for diagnostic, analytical, or treatment planning purposes. The displaying may further comprise superposing the aligned first and second stacks. For example, the first stack may be opaque and the second stack may have a degree of transparency, e.g. 50% transparency. The method may thereby enable the analysis of the evolution of a portion of the human body tissue by comparing the differences between the first and second stacks. Additionally or alternatively, the method may comprise storing the aligned first and second stacks. The first and second stacks may be stored in a digital format, e.g., in a non-volatile storage, a database or a cloud-based storage system. The storage may include metadata associated with the alignment process, such as timestamps.

The method thus improves the subsequent retrieval and review of the aligned first and second stacks, thereby improving decision making in a medical context.

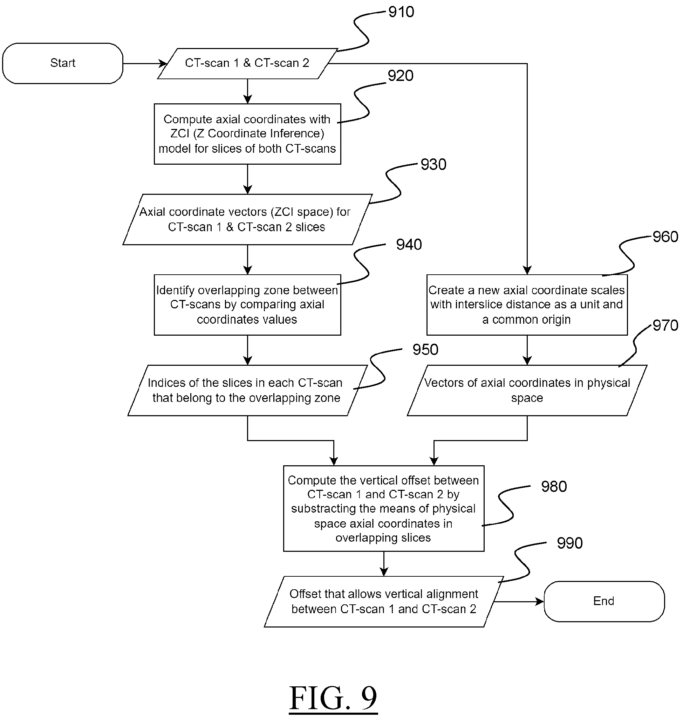

FIG. 9 illustrates a flowchart of the method.

The method may start 910 obtaining S10 a first stack of slices denoted as CT-scan 1 stack and a second stack of slices denoted as CT scan 2.

As a next step 920, the method computes axial coordinates with the neural network (denoted as ZCl or Z coordinate inference) and obtains 930 sets of axial coordinate vectors (represented in an ZCl space) for the slices of the first stack and the second stack. In other words, the method performs the computation S20.

The method identifies 940 overlapping zones between CT-scans by comparing axial coordinate values and sets 950 indices of the slices in each stack that belong to an overlapping zone. In other words, the method performs the computation S30.

The method also creates new axial coordinate scales (that is the new physical scales) with interslice space as unit and a common origin 960 and obtains vectors of axial coordinates in the new physical scales. In other words, the method performs the computation S40.

In this example flowchart, the computation S40 and S50 may be performed in parallel from the computations S20 and S30.

The method also computes the vertical offset between the slices of the first and second tack, CT-scan 1 and CT-scan 2 by subtracting the means of the new physical scale 980. In other words, the method performs the computation S60.

The method next obtains the offset that allows the vertical alignment of the first and second stacks 990. In other words, the method performs the computation S70.

## Claims

1. A computer implemented method for aligning a first and a second stack of slices along a common reference axis, each slice being a medical image, the common reference axis being normal to each slice of the first and second stacks, the method comprising:
- obtaining (S10) the first and the second stack of slices, each stack having a predetermined interslice space;
- computing (S20), for each stack, a set of coordinates lying in a scale common to the first and second stacks, each coordinate being associated, in the common reference axis, with a slice;
- computing (S30) an intersection between the two sets of coordinates, thereby identifying overlapping slices having coordinates which lie in the intersection;
- creating (S40), for each stack, a new physical scale of coordinates sharing a common origin, wherein two consecutive coordinates of the new physical scale are spaced by the predetermined interslice space;
- computing (S50), for each stack, the mean of the new physical scale of coordinates associated to overlapping slices;
- subtracting (S60) the mean computed for the second stack to the mean computed for the first stack, thereby obtaining a vertical offset along the common reference axis; and
- aligning (S70) the first and the second stacks of slices by offsetting the second stack of slices with the obtained vertical offset.

2. The method of claim 1, wherein computing (S20) the set of coordinates comprises:
- obtaining a trained neural network, the neural network being configured to take as input a pair of stacks of slices and to output a set of coordinates lying in a scale common to the pair of stacks, each coordinate being associated, in the common reference axis, with a slice;
- inputting the first and the second stack of slices to the trained neural network; and
- outputting the set of coordinates from the neural network.

3. The method of claim 2, wherein the trained neural network has been trained according to a machine-learning method comprising:
- providing a dataset of stacks of slices; and
- training the neural network based on the dataset, the training comprising providing as input a pair of stacks of slices from the dataset and regressing a slice score for each slice of the stack.

4. The method of claim 3, wherein regressing the slice score comprises minimizing a loss, the loss rewarding, for each slice score of a respective slice of the stack, at least one of:
- an ordering of each slice score;
- attributing a regular spacing between pairs of slice scores;
- resembling to straight lines.

5. The method of any one of claims 1 to 4, wherein creating (S40), for each stack, the new physical scale of coordinates sharing the common origin comprises setting a respective set of new coordinates, a new coordinate of the respective set corresponding to the common origin added to an index of a respective slice of the stack multiplied by an interslice space for the stack.

6. The method of claim 5, wherein setting the set of coordinates is performed, for a respective slice of each stack according to the formula: $∀ i ∈ \left[1, {N}_{1}\right] , {z}_{i} = {z}_{0} + i \times {δ}_{{z}_{1}}$ $∀ j ∈ \left[1, {N}_{2}\right] , {z}_{j} = {z}_{0} + j \times {δ}_{{z}_{2}}$ wherein *N*₁, *N*₂ denote respectively the number of slices in the first stack and the second stack, i denotes an index of a slice of the first stack, j denotes an index of a slice of the second stack, *z*₀ denotes the common origin of the physical scale for each volume, and *δ*_{*z*₁}, *δ*_{*z*₂} denote respectively an interslice space for the first stack and the second stack.

7. The method of claims 6, wherein computing (S50) the mean of the new physical scale of coordinates for each stack is performed according to the formula: $\overline{{Z}_{1}} = \frac{1}{Card \left({O}_{1}\right)} {\sum }_{{z}_{i} ∈ {O}_{1}} {z}_{i}$ $\overline{{Z}_{2}} = \frac{1}{Card \left({O}_{2}\right)} {\sum }_{{z}_{i} ∈ {O}_{2}} {z}_{i}$ wherein *̅Z̅*̅₁̅ denotes the mean of the new scale of coordinates for the first stack, *̅Z̅*̅₂̅ denotes the mean of the new scale of coordinates for the second stack, *O*₁ denotes the set of new coordinates for the new physical scale of coordinates for the first stack and *O*₂ denotes the set of new coordinates for the new physical scale of coordinates for the second stack.

8. The method of claim 7, wherein obtaining the vertical offset along the common reference axis is performed according to the formula: $Δ {Z}_{2 \to 1} = \overline{{Z}_{1}} - \overline{{Z}_{2}} ,$ wherein Δ*Z*_{2→1} denotes the vertical offset.

9. The method of any one of claims 1 to 8, further comprising applying a vertical translation to the slices of the first stack and the second stack, the vertical translation having a norm equal to the vertical offset.

10. The method of any one of claims 1 to 9, wherein each slice is a CT-Scan or an MRI or a PET Scan or SPECT Scan.

11. The method of any one of claims 1 to 10, further comprising displaying and/or storing the aligned first and the second stacks of slices.

12. A computer program comprising instructions which, when the program is executed by a computer, cause the computer to carry out the method of any of claims 1 to 11.

13. A computer readable storage medium having recorded thereon the computer program of claim 12.

14. A system comprising a processor communicatively coupled to a memory, the memory having recorded thereon the computer program of claim 12.
